# EUROPEAN PATENT APPLICATION

(11) **EP 4 356 846 A1**
(43) Date of publication of application: **24.04.2024**
(21) Application number: 22824322.6
(22) Date of filing: 17.06.2022
(51) Int. Cl.: A61B 17/02

(54) **ANTI-TORSION BENDING BALLOON DEVICE**

(30) Priority: 18.06.2021 CN 202110683384
(71) Applicant: Shanghai Keci Medical Technology Co., Ltd, Shanghai 201613 (CN)
(72) Inventor: RU, Chengtao, Shanghai 200135 (CN); ZHANG, Zhichao, Shanghai 200135 (CN)
(74) Representative: Cameron Intellectual Property Ltd
(86) International application number: PCT/CN2022/099480
(87) International publication number: WO 2022/262850

(57) **Abstract**

The present invention provides a torque-resistant curved balloon device, comprising a curved balloon and a kink-resistant fixing strip, wherein the curved balloon bends to one side after being inflated, and the inner curve of the curved balloon is connected to the fixing strip by means of a plurality of points or a whole section. The present invention strengthens the bending force provided by the curved balloon when bending, and enhances the torque resistance of the curved balloon, so that the curved balloon can bend more stably in accordance with the expected way during the bending.

## Description

### TECHNICAL FIELD

The present invention relates to the field of medical devices, especially to a torque-resistant curved balloon device.

### BACKGROUND

The general structure of a curved balloon catheter is a curved balloon and an internal core tube. The core tube has two openings at both ends of the balloon and goes through the balloon. The core tube in the balloon has an opening that can inflate the balloon with water or air. The connection between the curved balloon and the core tube is generally at the openings at both ends of the curved balloon, and the balloon and the core tube are connected and kept airtight by welding, bonding or other connection techniques. However, the core tube is generally free to move relative to the balloon in the middle part of the balloon, that is, the core tube is not fixed to the balloon in the middle part of the balloon.

The most basic principle of the curved balloon is that the elongation of the material on the outer bending side of the curved balloon is greater than the elongation of the material on the opposite side, thereby causing the balloon to bend. The general curved balloon relies on the structure of the curved balloon to cause different elongations on the inner and outer sides to bend, and also relies on the core tube on the inner side of the curved balloon as a support with a constant length, because the core tube material is different from the balloon, the core tube cannot be stretched, and provides a constant length effect on the inner side of the curved balloon.

However, it is found in actual use that after the curved balloon is bent, a reverse bending force or a twisting force is applied to the curved balloon, causing the curved balloon to deform. Since the position of the core tube is not fixed relative to the balloon, the position of the core tube deviates. In the bent shape, the core tube changes its position as a segment with a certain length, thereby completely changing the bent shape. From the perspective of mechanical performance, such a curved balloon has poor bending and twisting resistance.

Therefore, there is a need to provide a new curved balloon device that enhances the bending force and torque resistance of the curved balloon, so that the curved balloon can bend more stably in accordance with the expected way during the bending process.

### Invention content

The object of the present invention is to solve the defect of poor bending and twisting resistance of the existing curved balloon, and to provide a new curved balloon device that enhances the bending strength and torque resistance of the curved balloon.

To achieve the above object, the present invention provides a torque-resistant curved balloon device, comprising a curved balloon and a kink-resistant fixing strip, wherein the curved balloon bends to one side after being inflated, and the inner curve of the curved balloon is connected to the fixing strip by means of a plurality of points or a whole section.

Wherein, the fixing strip is a core tube, the core tube has an opening that can inflate the balloon with water or air. The core tube is located within the curved balloon, and the two ends together with several points or one-segment of the core tube are connected and fixed to the inner curve of the curved balloon.

Particularly, the core tube can be outside the curved balloon, and has an opening that communicates with the balloon, and several points or one-segment connection of the core tube are connected and fixed to the inner curve of the curved balloon.

Preferably, the fixing strip is a reinforcing rib, the reinforcing rib is outside the curved balloon, the inside of the curved balloon is provided with a core tube, the two ends of the core tube are sealed and connected to the curved balloon, the core tube has an opening that can inflate the balloon with water or air.

The fixing strip is fixedly connected to the curved balloon by welding. The fixing strip can also be fixedly connected to the curved balloon by glue bonding. The fixing strip is fixedly connected to the curved balloon by whole-line connection. The fixing strip can also be fixedly connected to the curved balloon by means of a plurality of points.

The beneficial effect of the present invention is to provide a new curved balloon device that strengthens the bending force provided by the curved balloon when bending, and enhances the torque resistance of the curved balloon, so that the curved balloon can bend more stably in accordance with the expected way during the bending process.

In order to make the above-mentioned objects, features and advantages of the present invention more obvious and understandable, the following preferred embodiments are given, and the accompanying drawings are used for detailed explanation as follows.

### ILLUSTRATE WITH DIAGRAMS

In order to more clearly illustrate the technical solutions in the embodiments of the present invention or in the prior art, the drawings needed to be used in the description of the embodiments or the prior art will be briefly introduced below. Obviously, the drawings in the following description are only some embodiments of the present invention, and not all embodiments. For a person having ordinary skill in the art, other drawings can be obtained without creative efforts based on these drawings.
FIG. 1 is a schematic diagram of an embodiment of the present invention;
FIG. 2 is a schematic cross-sectional view of the middle section of the embodiment of FIG. 1;
FIG. 3 is a schematic diagram of another embodiment of the present invention;
FIG. 4 is a schematic diagram of yet another embodiment of the present invention.

### SPECIFIC EMBODIMENTS

The technical solutions in the embodiments of the present invention will be clearly and completely described below with reference to the drawings in the embodiments of the present invention. Obviously, the described embodiments are only a part of the embodiments of the present invention, and not all embodiments. Usually, the components of the embodiments of the present invention shown and described in the accompanying drawings can be arranged and designed in various different configurations. Therefore, the following detailed description of the embodiments of the present invention provided in the accompanying drawings is not intended to limit the scope of the present invention claimed, but merely represents the selected embodiments of the present invention. Based on the embodiments of the present invention, all other embodiments obtained by those skilled in the art without making creative efforts belong to the scope of protection of the present invention.

The present invention will be further described in detail below to enable those skilled in the art to refer to the specification and implement it.

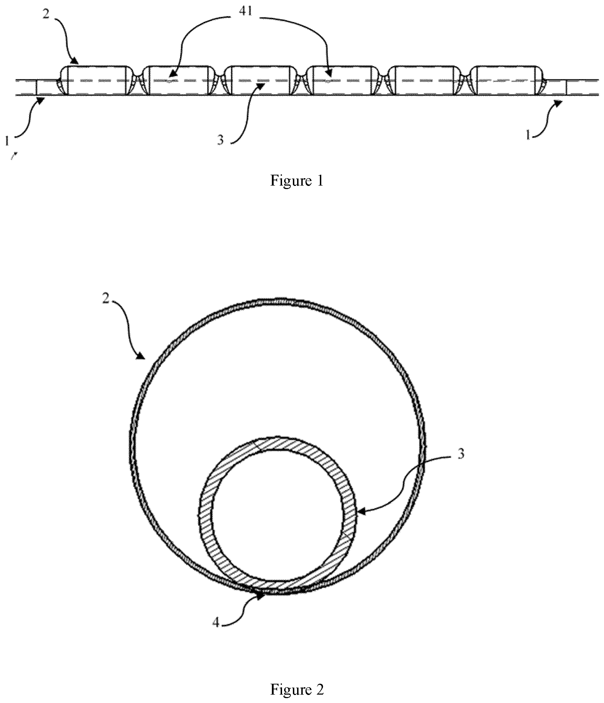

First, please refer to FIG. 1, which is a schematic diagram of the present invention. In FIG. 1, the fixing strip with anti-torque property is a core tube 3, which is generally made of plastic or polymer material, has a certain flexibility, but does not have obvious ductility, can be bent but has a certain strength.

The core tube 3 passes through the curved balloon 2, and the curved balloon 2 and the core tube 3 are connected at both ends 1. The core tube has an opening 41 that can inflate the balloon with water or air. The core tube 3 is connected to the inner curve of the curved balloon 2 by a whole line 4. With such a structure, when the curved balloon 2 is inflated, due to the characteristic of the curved balloon bending to one side, the outer side of the curved balloon 2 expands more than the inner side, and the curved balloon 2 forms a C-shaped inward bend, thereby forming a certain displacement that can pull the human body cavity tube where it is located. However, since the balloon itself is soft, it is not easy to shape, has a small pulling force, and is prone to form a twisted shape that cannot achieve the desired pulling effect.

The core tube 3 has a certain strength, which is not easy to deform and has anti-torque property, it is connected to the inner curve of the curved balloon 2 by a whole line 4. It is equivalent to fixing the position and length of the inner curve of the curved balloon 2, thereby making the entire curved balloon 2 not easy to stretch and twist, and can achieve the preset shape. At the same time, since the core tube 3 has a certain strength, the inflated curved balloon 3 has a certain pulling force.

In some other embodiments, the core tube 3 is connected to the inner curve of the curved balloon 2 by means of a plurality of points or one segment. The connection between the core tube 3 and the curved balloon 2 in the middle section can be glue bonding or plastic welding.

Next, please refer to FIG. 2, which is a schematic cross-sectional view of the middle section of the present invention. The core tube 3 passes through the middle of the curved balloon 2 and is connected and fixed at the connection 4, and the connection 4 is located at the inner curve of the curved balloon 2.

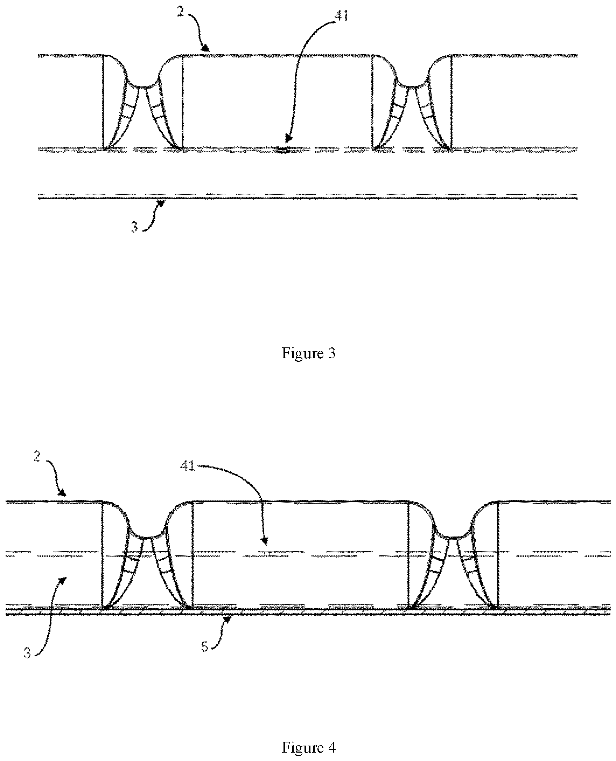

As shown in FIG. 3, in some embodiments, the core tube 3 is set outside the curved balloon 2 and is on the inner curve of the curved balloon 2. The curved balloon 2 and the core tube 3 are connected by means of a plurality of points or one segment, and have an opening 41 that can inflate the curved balloon 2 with water or air.

As shown in FIG. 4, particularly, in some embodiments, the fixing strip is a reinforcing rib 5, which is located at the inner curve of the curved balloon 2 and does not have ductility. The inside of the curved balloon 2 is provided with a core tube 3, and the two ends of the core tube 3 are sealed and connected to the curved balloon 2 itself, and the core tube 3 has an opening 41 that can inflate the balloon with water or air.

Although the embodiments of the present invention have been disclosed as above, they are not intended to limit the present invention. They can be applied to various fields suitable for the present invention. For those skilled in the art, other modifications can be easily made without creative efforts. Therefore, the present invention is not limited to the specific details and illustrations shown and described herein. Within the general concept of the claims and the equivalent scope, the present invention covers all other embodiments.

## Claims

1. A torque-resistant curved balloon device, comprising a curved balloon and a kink-resistant fixing strip, wherein the curved balloon bends to one side after being inflated, **characterized in that** the inner curve of the curved balloon is connected to the fixing strip by means of a plurality of points or a whole section.

2. The torque-resistant curved balloon device according to claim 1, **characterized in that** the fixing strip is a core tube, the core tube has an opening that can inflate the balloon with water or air.

3. The torque-resistant curved balloon device according to claim 2, **characterized in that** the core tube is located within the curved balloon, and the two ends together with several points or one-segment connection of the core tube are connected and fixed to the inner curve of the curved balloon.

4. The torque-resistant curved balloon device according to claim 2, **characterized in that** the core tube is outside the curved balloon, and has an opening that communicates with the balloon, and several points or one-segment connection of the core tube are connected and fixed to the inner curve of the curved balloon.

5. The torque-resistant curved balloon device according to claim 1, **characterized in that** the fixing strip is a reinforcing rib, the reinforcing rib is outside the curved balloon, the inside of the curved balloon is provided with a core tube, the two ends of the core tube are sealed and connected to the curved balloon, the core tube has an opening that can inflate the balloon with water or air.

6. The torque-resistant curved balloon device according to claim 1, **characterized in that** the fixing strip is fixedly connected to the curved balloon by welding.

7. The torque-resistant curved balloon device according to claim 1, **characterized in that** the fixing strip is fixedly connected to the curved balloon by glue bonding.

8. The torque-resistant curved balloon device according to claim 1, **characterized in that** the fixing strip is fixedly connected to the curved balloon by means of one-segment connection.

9. The torque-resistant curved balloon device according to claim 1, **characterized in that** the fixing strip is fixedly connected to the curved balloon by means of a plurality of points.
